# EUROPEAN PATENT SPECIFICATION

(11) **EP 3 533 869 B1**
(45) Date of publication and mention of the grant of the patent: **20.08.2025**
(21) Application number: 17865540.3
(22) Date of filing: 12.10.2017
(51) Int. Cl.: C12N 15/09, C12N 9/10, C12Q 1/68

(54) **MUTANT HUMAN DNA POLYMERASE**
MUTIERTE MENSCHLICHE DNA-POLYMERASE
ADN POLYMÉRASE EPSILON HUMAINE MUTANTE

(30) Priority: 26.10.2016 JP 2016209688
(43) Date of publication of application: 04.09.2019
(73) Proprietor: Denka Company Limited, Tokyo 103-8338 (JP); Niigata University, Niigata-shi Niigata 950-2181 (JP)
(72) Inventor: IZUTSU, Hiroshi, Machida-city Tokyo 194-8560 (JP); KODAMA, Keisuke, Machida-city Tokyo 194-8560 (JP); NAKADA, Mitsutaka, Machida-city Tokyo 194-8560 (JP); WAKAI, Toshifumi, Niigata-shi Niigata 951-8510 (JP); KAMEYAMA, Hitoshi, Niigata-shi Niigata 951-8510 (JP); NAGAHASHI, Masayuki, Niigata-shi Niigata 951-8510 (JP); SHIMADA, Yoshifumi, Niigata-shi Niigata 951-8510 (JP); ICHIKAWA, Hiroshi, Niigata-shi Niigata 951-8510 (JP)
(74) Representative: Jacob, Reuben Ellis
(86) International application number: PCT/JP2017/037039
(87) International publication number: WO 2018/079287

(56) References cited:
- WO-A1-2015/112930
- WO-A1-2015/112930
- SIRIPAN LIMSIRICHAIKUL ET AL: "The Gly-952 Residue of Saccharomyces cerevisiae DNA Polymerase Is Important in Discriminating Correct Deoxyribonucleotides from Incorrect Ones", 1 June 2003 (2003-06-01), pages 19079 - 19086, XP055689390, Retrieved from the Internet <URL:https://www.researchgate.net/publication/7968270_The_Gly-952_Residue_of_Saccharomyces_cerevisiae_DNA_Polymerase_Is_Important_in_Discriminating_Correct_Deoxyribonucleotides_from_Incorrect_Ones> [retrieved on 20200427], DOI: 10.1074/jbc.M208604200.Source:
- LIEN N. HOANG ET AL: "Domain Mutations in Ovarian Endometrioid Carcinoma", INTERNATIONAL JOURNAL OF GYNECOLOGICAL CANCER, vol. 25, no. 7, 1 September 2015 (2015-09-01), US, pages 1187 - 1193, XP055689476, ISSN: 1048-891X, DOI: 10.1097/IGC.0000000000000492
- STEVEN A ROBERTS ET AL: "Hypermutation in human cancer genomes: footprints and mechanisms", NATURE REVIEWS CANCER, 1 December 2014 (2014-12-01), pages 786 - 800, XP055689469, Retrieved from the Internet <URL:https://www.ncbi.nlm.nih.gov/pmc/articles/PMC4280484/pdf/nihms-648998.pdf> DOI: 10.1038/nrc3816
- CAROLINE C. BILLINGSLEY ET AL: "Polymerase [epsilon] ( POLE ) mutations in endometrial cancer: Clinical outcomes and implications for Lynch syndrome testing : POLE Mutations and Endometrial Cancer", CANCER., vol. 121, no. 3, 15 September 2014 (2014-09-15), US, pages 386 - 394, XP055661332, ISSN: 0008-543X, DOI: 10.1002/cncr.29046
- AHN SUNG-MIN ET AL: "The somatic POLE P286R mutation defines a unique subclass of colorectal cancer featuring hypermutation, representing a potential genomic biomarker for immunotherapy", ONCOTARGET,, vol. 7, no. 42, 18 October 2016 (2016-10-18), pages 68638 - 68649, XP002779915
- KESTI, T. ET AL.: "Molecular cloning of the cDNA for the catalytic subunit of human DNA polymerase epsilon", J. BIOL. CHEM., vol. 268, no. 14, 1993, pages 10238 - 10245, XP055595069
- SHLIEN, A. ET AL.: "Combined hereditary and somatic mutations of replication error repair genes result in rapid onset of ultra-hypermutated cancers", NATURE GENETICS, vol. 47, no. 3, 2015, pages 257 - 262, XP055595073
- "The Cancer Genome Atlas Network, Comprehensive molecular characterization of human colon and rectal cancer", NATURE, vol. 487, 2012, pages 330 - 337, XP055041570
- BRIGGS, S. ET AL.: "Germline and somatic polymerase epsilon and delta mutations define a new class of hypermutated colorectal and endometrial cancers", J. PATHOL., vol. 230, 2013, pages 148 - 153, XP055595079

## Description

### Technical Field

The present invention relates to a mutant human DNA polymerase ε. The present invention also relates to a nucleic acid encoding a mutant human DNA polymerase ε and a fragment thereof and a method for determining whether a cancer is a hypermutated cancer or not.

### Background Art

One of the patterns of the genetic mutation in cancer is hypermutation (hypermutated cancer). Hypermutated cancer is distinguished from other forms by higher somatic mutation rates than other forms. And it is known that cancers that exhibit hypermutation characteristics are found in gastric cancer, breast cancer, colorectal cancer, glioblastoma, uterine carcinoma, and the like (Non Patent Literature 1).

Hypermutated cancer often has properties of microsatellite instability, which indicates deficient or imperfection of the mismatch repair mechanism in DNA replication. This is considered to be caused by mutation of one or more of the genes of the mismatch repair enzymes MLH1, MLH3, MSH2, MSH3, MSH6, and PMS2, suppression of the expression of the MLH1 gene by methylation, or the like.

Moreover, mutation of DNA polymerase ε, which is a DNA replicative enzyme, is known to cause somatic mutation at particularly high frequencies and lead to hypermutation (Non Patent Literature 2). DNA polymerase ε has, in addition to 5' → 3' direction DNA-synthesizing activity, 3' → 5' exonuclease activity as an error correction mechanism and, when an incorrect nucleotide is added to a DNA strand, removes the nucleotide by the 3' → 5' exonuclease activity, which is followed by resumption of DNA synthesis. Therefore, the association between hypermutated cancer and DNA polymerase ε is considered to be caused by loss of normal function of the error correction mechanism of DNA polymerase ε and the association between mutation in the exonuclease activity domain (the positions 87 to 426 in the amino acid sequence) and hypermutation has been examined.

For example, mutations such as D275V, P286R, S297F, H342R, F367S, V411L, L424V, A426V, and S459F have been disclosed as mutations of amino acid residues in DNA polymerase ε that causes hypermutation (Non Patent Literatures 3 to 7).

Cancer immune escape mechanisms have been elucidated in recent years and new cancer immunotherapies targeting this mechanism has been clinically applied. Such mechanisms include the PD-1 (Programmed cell Death-1)/PD-L1 (PD-1 Ligand 1) pathway, which is also referred to as the immunocheckpoint pathway. Blockage of the PD-1/PD-L1 pathway, which transmits immune suppressive support signals, cancels the suppression of T cell immunity to activate T cells and suppresses tumor expressing one or more cancer-specific antigens (Patent Literature 1). Moreover, CTLA-4 is also expressed in activated T cells and binding of CD28 ligand on antigen presenting cells suppresses the activation of the T cells and therefore it is also possible to cancel the immunosuppression of T cells and cause tumor suppression by blocking this pathway (Non Patent Literature 8). Anti-cancer agents based on this principle have been in practical use (e.g., nivolumab, ipilimumab) (Non Patent Literatures 9 to 12). Furthermore, it is expected that there should be a plurality of other immunosuppression mechanisms and antitumor agents that block these immunosuppression mechanisms would be developed and put into practical use in the future.

Hypermutated cancers have many cancer-specific antigens that can be targeted by the immune mechanism and therapies that block immunosuppression signal pathways have been shown to be effective on hypermutated cancers (Non-Patent Literatures 13 to 14). Therefore, a biomarker that makes it possible to determine that a cancer is hypermutated is useful, for example, in the selection of an appropriate therapy, the determination of therapy policy, and the like.

### Citation List

### Patent Literature

Patent Literature 1: Japanese Patent No. 4409430

### Non Patent Literature

Non Patent Literature 1: Nat. Rev. Cancer, 2014, Vol. 14 (12), pp. 786-800
Non Patent Literature 2: J. Pathol., 2013, Vol. 230, pp. 148-153
Non Patent Literature 3: Int. J. Gynecol. Cancer, 2015, Vol. 25 (7), pp. 1187-1193
Non Patent Literature 4: J. Pathol., 2013, Vol. 230, pp. 148-153
Non Patent Literature 5: Cancer, 2015, Vol. 121 (3), pp. 386-394
Non Patent Literature 6: Clin. Cancer Res., 2015, Vol. 21 (14), pp. 3347-3355
Non Patent Literature 7: European Journal of Human Genetics, 2011, Vol. 19, pp. 320-325
Non Patent Literature 8: Annals of The Japanese Respiratory Society, 2014, Vol. 3 (1), pp. 43-49
Non Patent Literature 9: International Immunology, 2007, Vol. 19 (7), pp. 813-824
Non Patent Literature 10: Cancer Immunol. Res., 2014, Vol. 2 (9), pp. 846-856
Non Patent Literature 11: The New England Journal of Medicine, 2010, Vol. 363 (8), pp. 711-723
Non Patent Literature 12: Cancer Cell, 2015, Vol. 27 (4), pp. 439-449
Non Patent Literature 13: Science, 2015, Vol. 348 (6230), pp. 124-128
Non Patent Literature 14: The New England Journal of Medicine, 2015, Vol. 372 (26), pp. 2509-2520
Non Patent Literature 15: Journal of Biological Chemistry, 2003, Vol. 278 (21), pp.19079-19086

### Summary of Invention

### Problems to be Solved by the Invention

An object of the present invention is to provide a new biomarker that makes it possible to distinguish hypermutated cancer.

### Means for Solving the Problems

According to the invention there is provided a mutant human DNA polymerase ε according to SEQ ID NO: 1 comprising mutation at amino acid position 952, wherein the mutation comprises mutation of a leucine residue at the position 952 in the amino acid sequence of human DNA polymerase ε into another amino acid residue wherein the other amino acid residue is a serine residue. The present inventors have found a mutation of the amino acid residue at the position 952 in the DNA synthesis activity domain (the positions 624 to 1145 in the amino acid sequence) in DNA polymerase ε in a hypermutated cancer. The association between hypermutated cancer and mutation of amino acid residues in the exonuclease activity domain in DNA polymerase ε has been known so far. Meanwhile, the association between hypermutated cancer and mutation of amino acid residues in the DNA synthesis activity domain in DNA polymerase ε has not been known so far. The present invention is based on these novel findings.

The present invention provides a mutant human DNA polymerase ε comprising mutation of one or more amino acids, wherein the mutation of one or more amino acids comprises mutation of a leucine residue at the position 952 in the amino acid sequence of human DNA polymerase ε into another amino acid residue.

The mutant human DNA polymerase ε according to the present invention is found in hypermutated cancer and therefore useful, for example, as a new biomarker for distinguishing hypermutated cancer.

The other amino acid residue aforementioned may be any amino acid residue other than a leucine residue, but it is preferred to be a serine residue. To be a serine residue increases determination accuracy in determination of hypermutated cancer.

The present invention relates to a nucleic acid encoding the mutant according to the present invention. Moreover, the present invention relates to a nucleic acid complementary to a nucleic acid encoding the mutant human DNA polymerase ε according to the present invention. The nucleic acid or complementary nucleic acid may be a fragment comprising a nucleotide sequence (codon sequence) encoding the other amino acid residue aforementioned or a complementary sequence thereof. The fragment is also a nucleic acid and the nucleotide length thereof may be, for example, 20 to 300 nucleotide long.

The present invention also provides a method for determining whether a cancer is a hypermutated cancer or not in a human patient with cancer, comprising determining whether mutation is present or absent at an amino acid residue at the position 952 in the amino acid sequence of human DNA polymerase ε derived from a cancer cell taken from the human patient using a sample comprising one or more components derived from the cancer cell, wherein the presence of mutation at the amino acid residue indicates that the cancer is a hypermutated cancer.

According to the method according to the present invention, whether a cancer is a hypermutated cancer can be determined in a human patient with cancer.

In the method according to the present invention, the determination of presence or absence of mutation at the amino acid residue may comprise detection of mutation in a nucleotide sequence encoding the amino acid residue at the position 952 in the amino acid sequence of human DNA polymerase ε.

In the method according to the present invention, it is preferable that the mutation at the aforementioned amino acid residue be L952S. This increases the determination accuracy.

In the method according to the present invention, the aforementioned cancer may be colorectal cancer or rectal cancer. When the cancer is colorectal cancer or rectal cancer, whether the cancer is a hypermutated cancer or not can be determined at higher certainty.

The present invention also provides a method for diagnosing hypermutated cancer, comprising detecting mutation at an amino acid residue at the position 952 in the amino acid sequence of human DNA polymerase ε derived from a cell taken from a human specimen using a sample comprising one or more components derived from the cell, wherein if the amino acid residue is mutated, the human specimen is diagnosed as having hypermutated cancer.

### Effects of the Invention

According to the present invention, a new biomarker that makes it possible to distinguish hypermutated cancer is provided.

### Brief Description of Drawing

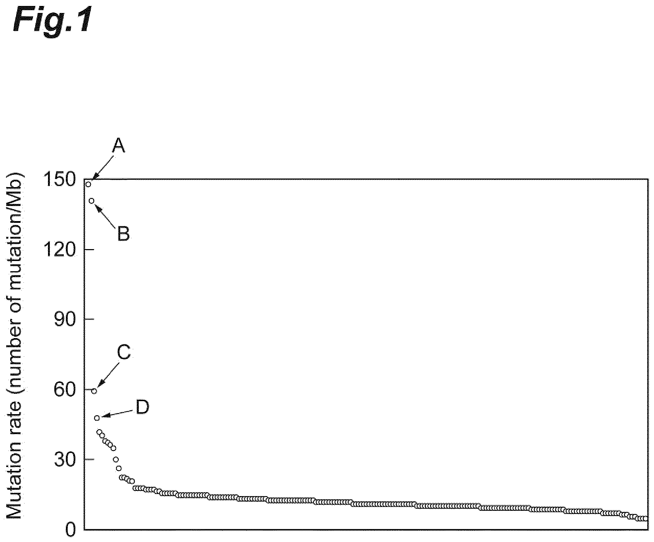

[Figure 1] Figure 1 is a graph illustrating the results of Examples.

### Embodiments for Carrying Out the Invention

Modes for carrying out the present invention will be described in detail below. The present invention is not limited to the following embodiment.

### [Hypermutated cancer]

Hypermutated cancer is cancer having a higher mutation rate of cancer specific mutations than usual cancer. Here, the mutation rate is the number of mutations associated with change of amino acid residues per 10⁶ bases (hereinafter, also referred to as "Mb"). Hypermutated cancer is determined by the comparison of the mutation rate with that of usual cancer. Although the mutation rate varies depending on the number and kind of genes examined as well as the kind of cancer, the cancer is determined to be a hypermutated cancer if the mutation rate of a cancer is relatively higher than that of usual cancer.

### [Human DNA polymerase ε]

Human DNA polymerase ε is a DNA replicative enzyme and the wild type DNA polymerase ε has the amino acid sequence set forth in SEQ ID NO: 1. The DNA synthesis activity domain of human DNA polymerase ε corresponds to the positions 624 to 1145 of the amino acid sequence set forth in SEQ ID NO: 1 and the exonuclease activity domain corresponds to the positions 87 to 426 of the amino acid sequence set forth in SEQ ID NO: 1.

As used herein, the mutation at an amino acid residue means that a particular amino acid residue in the amino acid sequence of a protein is changed to an amino acid residue different from the amino acid residue in the corresponding wild type amino acid sequence. The mutation at an amino acid residue may be any of substitution of, deletion of, or insertion to the amino acid residue. For example, the amino acid residue at the position 952 of the amino acid sequence of the wild type human DNA polymerase ε set forth in SEQ ID NO: 1 is leucine and mutation is present when this is changed to an amino acid residue other than leucine.

The mutant human DNA polymerase ε according to the present embodiment comprises mutation of one or more amino acid residues and the mutation of one or more amino acid residues comprises at least mutation of a leucine residue at the position 952 in the amino acid sequence of human DNA polymerase ε into another amino acid residue.

Here, another amino acid residue is not particularly limited, as long as it is an amino acid residue other than a leucine residue, but it may be, for example, a serine residue, a methionine residue, a valine residue, a tryptophan residue, or a phenylalanine residue.

It is preferable that the mutant human DNA polymerase ε according to the present embodiment comprise mutation in which at least a leucine residue at the position 952 in the amino acid sequence of human DNA polymerase ε is substituted with another amino acid residue, and more preferable that it comprise mutation (L952S) in which at least a leucine residue at the position 952 in the amino acid sequence of human DNA polymerase ε is substituted with a serine residue. This increases the determination accuracy when it is used as a biomarker for distinguishing hypermutated cancer.

The mutant human DNA polymerase ε according to the present embodiment may be a mutant comprising only L952S mutation (SEQ ID NO: 2) or may be a mutant comprising mutation of one or more further amino acid residues other than the L952S mutation.

Examples of the mutation of one or more amino acid residues that can be comprised other than the L952S mutation include mutations such as D275V, P286R, S297F, H342R, F367S, V411L, L424V, A426V, and S459F.

The mutant human DNA polymerase ε according to the present embodiment may be obtained by extraction and purification from human cancer cells having the mutant or may be obtained by obtaining a nucleic acid encoding the mutant human DNA polymerase ε by chemical synthesis or the like and then expressing the nucleic acid in an appropriate protein expression system.

### [Nucleic acid]

The nucleic acid according to one embodiment is a nucleic acid (hereinafter, also referred to as "Nucleic acid 1") encoding the aforementioned mutant human DNA polymerase ε. Nucleic acid 1 may be a nucleic acid having a genomic DNA sequence including intron or may be a nucleic acid having an mRNA sequence. The genomic DNA sequence of the wild type human DNA polymerase ε gene is NCBI accession number NG_033840. The mRNA sequence of the wild type human DNA polymerase ε is NCBI accession number L09561.

A nucleic acid according to another embodiment is a nucleic acid (hereinafter, also referred to as "Nucleic acid 2") having the nucleotide sequence complementary to Nucleic acid 1.

In one embodiment, the nucleic acid may be a fragment of Nucleic acid 1 and the fragment comprises a nucleotide sequence (codon sequence) encoding the other amino acid residue at the position 952 in the amino acid sequence of the mutant human DNA polymerase ε mentioned above (hereinafter, the nucleic acid of this fragment is also referred to as "Nucleic acid 3").

In a further embodiment, the nucleic acid may be a fragment of Nucleic acid 2 and the fragment comprises the complementary sequence of a nucleotide sequence (codon sequence) encoding the other amino acid residue at the position 952 in the amino acid sequence of the aforementioned mutant human DNA polymerase ε (hereinafter, the nucleic acid of this fragment is also referred to as "Nucleic acid 4").

Nucleic acid 1 to Nucleic acid 4 may be used as a biomarker for distinguishing hypermutated cancer. More specifically, mutation at an amino acid residue in the mutant human DNA polymerase ε can be detected, for example, by detecting mutation in the nucleotide sequences of Nucleic acid 1 to Nucleic acid 4. Nucleic acid 1 to Nucleic acid 4 may be a nucleic acid having a cDNA nucleotide sequence in which intron is removed from the genomic DNA sequence.

As used herein, the "mutation in the nucleotide sequence" means that at least a part of the nucleotides in the nucleotide sequence is substituted with one or more other nucleotides such that one or more amino acid residues encoded by the nucleotide sequence are different from the amino acid residue(s) encoded by the corresponding wild type nucleotide sequence (also referred to as "missense mutation").

Moreover, Nucleic acid 1 to Nucleic acid 4 may be used as a probe for detecting mutation in the nucleotide sequence, for example, by cloning into an appropriate cloning vector.

The nucleotide length of Nucleic acid 3 and Nucleic acid 4 (fragments) is not particularly limited, but it is preferable that the nucleotide length of Nucleic acid 3 and Nucleic acid 4 be 20 to 300 nucleotide long, and it is more preferably 50 to 250 nucleotide long, further preferably 70 to 200 nucleotide long, and even more preferably 100 to 200 nucleotide long, in view of, for example, making it possible to detect more efficiently the mutation in the nucleotide sequence with a next-generation sequencer.

Moreover, it is preferable that Nucleic acid 3 and Nucleic acid 4 (fragments) be a nucleic acid encoding at least 7 consecutive amino acid residues including the "other amino acid residue" as stated above, more preferable that Nucleic acid 3 and Nucleic acid 4 (fragments) be a nucleic acid encoding at least 10 consecutive amino acid residues including the "other amino acid residue", in view of, for example, making it possible to detect more efficiently the mutation in the nucleotide sequence with a next-generation sequencer.

### [Method for determining whether a cancer is a hypermutated cancer or not]

The method for determination according to the present embodiment is a method for determining whether a cancer is a hypermutated cancer or not in a human patient with cancer, comprising determining whether an amino acid residue at the position 952 in the amino acid sequence of human DNA polymerase ε derived from a cancer cell taken from the human patient is mutated or not using a sample comprising one or more components derived from the cancer cell, wherein the presence of mutation at the amino acid residue indicates that the cancer is a hypermutated cancer.

The sample comprising one or more components derived from a cancer cell refers to a sample comprising one or more of the cancer cell itself, the nucleus of the cancer cell, the cytoplasm of the cancer cell, protein derived from the cancer cell, peptide derived from the cancer cell, and nucleic acid derived from the cancer cell. Examples of the sample comprising one or more components derived from a cancer cell include a cancer excision tissue specimen, a biopsy specimen, cancer cells that have infiltrated into ascitic fluid, circulating cancer cells, serum, plasma, blood, feces, urine, a sputum, spinal fluid, pleural fluid, nipple aspirate fluid, lymph, a cell culture medium, and other tissue and fluid taken from a patient. From the viewpoint of distinguishing hypermutated cancer at a higher certainty, it is preferable that the sample comprising one or more components derived from a cancer cell be a cancer excision tissue specimen, a biopsy specimen, cancer cells that have infiltrated an ascitic fluid, circulating cancer cells, serum, or plasma and more preferable that it be a cancer excision tissue specimen or a biopsy specimen. Moreover, when the sample comprising one or more components derived from a cancer cell is a cancer excision tissue specimen or a biopsy specimen, these specimens may be processed by a treatment, such as freezing, alcohol fixation, formalin fixation, paraffin embedding, or a combination thereof.

The mutation of the amino acid residue at the position 952 in the amino acid sequence of human DNA polymerase ε may be a mutation of a leucine residue into a serine residue, a methionine residue, a valine residue, a tryptophan residue, or a phenylalanine residue. When the mutation of the amino acid residue is one of these mutations, hypermutated cancer can be determined in a higher certainty. When the mutation is a mutation in which the leucine residue is substituted with a serine residue (L952S) among them, hypermutated cancer can be determined at an even higher certainty.

The detection of mutation at an amino acid residue may be performed by a known method. The detection of mutation may comprise, for example, the detection of mutation in a nucleotide sequence encoding the amino acid residue at the position 952 in the amino acid sequence of human DNA polymerase ε.

The detection of mutation in the nucleotide sequence may be performed by a known method. The detection of mutation in the nucleotide sequence may be performed, for example, by DNA sequencing (for example, sequencing by a next-generation sequencer), polymerase chain reaction, allele-specific amplification, hybridization with an allele-specific probe, mismatch cleavage analysis, single-strand conformation polymorphism, denaturing gradient gel electrophoresis, or temperature gradient gel electrophoresis. These techniques may be used alone or a plurality of techniques may be used in combination.

If the amino acid residue at the position 952 in the amino acid sequence of human DNA polymerase ε in the cancer cell is mutated, then the cancer can be determined to be a hypermutated cancer.

Examples of the cancer to which the invention according to the present embodiment can be applied include colorectal cancer, rectal cancer, colon cancer, gastric cancer, liver cancer, thyroid cancer, uterine carcinoma, kidney cancer, pancreatic cancer, tongue cancer, prostate cancer, lung cancer, skin cancer, ovarian cancer, gallbladder cancer, head and neck carcinoma, testicular tumor, adrenal cancer, oral cancer, bone and soft tissue tumor, brain tumor, malignant melanoma, osteosarcoma, chondrosarcoma, rhabdomyosarcoma, leiomyosarcoma, leukemia, malignant lymphoma, and multiple myeloma. If the cancer is colorectal cancer or rectal cancer among them, then hypermutated cancer can be distinguished at a higher certainty.

### [Method for diagnosing hypermutated cancer]

The method for diagnosis according to the present embodiment is a method for diagnosing hypermutated cancer, comprising detecting mutation at an amino acid residue at the position 952 in the amino acid sequence of human DNA polymerase ε derived from a cell taken from a human specimen using a sample comprising one or more components derived from the cell, wherein if the amino acid residue is mutated, the human specimen is diagnosed as having hypermutated cancer.

The sample comprising one or more components derived from a cell refers to a sample comprising one or more of the cell itself, the nucleus of the cell, the cytoplasm of the cell, protein derived from the cell, peptide derived from the cell, and nucleic acid derived from the cell. Examples of the sample comprising one or more components derived from a cell include a biopsy specimen, serum, plasma, blood, feces, urine, a sputum, spinal fluid, pleural fluid, a nipple aspirate fluid, lymph, a cell culture medium, and other tissues and fluids taken from human. From the viewpoint of distinguishing hypermutated cancer at a higher certainty, the sample comprising one or more components derived from a cell may be a biopsy specimen, serum, or plasma. When the sample comprising one or more components derived from a cell is a biopsy specimen, the biopsy specimen may be processed by a treatment, such as freezing, alcohol fixation, formalin fixation, paraffin embedding, or a combination thereof.

In the aforementioned method for diagnosis, the mutation of amino acid residues and detection thereof and cancers to which the method for diagnosis can be applied, and the like may be as described above.

### Examples

The present invention will be described more specifically referring to Examples, below. However, the present invention is not limited by Examples below.

### (Sample preparation)

Formalin fixed, paraffin-embedded, cancer excision specimens from 201 colorectal cancer patients were prepared and sliced into 2 sections with a thickness of 20 µm. The 2 prepared sections applied onto a slide glass were used as a sample for DNA extraction.

Separately from the sample for DNA extraction, a section with a thickness 4 µm was prepared and the prepared section applied on to a slide glass was used as a sample for microscopy.

### (DNA extraction)

The sample for microscopy was stained with hematoxylin eosin. The sample after the staining was observed with a microscope and identified a site containing many cancer cells in the section. The site identified with the sample for microscopy was scraped from the sample for DNA extraction with a razor and DNA was extracted from the site scraped off. The extraction of DNA was performed using BiOstic (registered trademark) FFPE Tissue DNA Isolation Kit (trade name).

### (Sequence analysis)

The DNAs extracted from formalin fixed, paraffin-embedded, cancer excision specimens from the 201 colorectal cancer patients were analyzed for cancer-specific mutations (mutations associated with mutation of amino acid residues) of 415 genes with a next-generation sequencer (MiSeq sequencer, Illumina, Inc.). The result of the analysis is illustrated in Figure 1.

Figure 1 is a graph in which the mutation rate (the number of mutations per 1 Mb) of each specimen is plotted. On the abscissa of Figure 1, the specimens were lined up from those with higher mutation rates to those with lower mutation rates in the direction from left to right. As illustrated in Figure 1, many mutations were detected in 17 specimens and these specimens were considered to be hypermutated. Among the 17 specimens, the mutation rates of 4 specimens (the specimens indicated with A to D in Figure 1) were markedly higher than the others. And, no mutation was found in mismatch repair genes in the specimens indicated with A, B, and D in Figure 1. To investigate this cause, these 4 specimens were further analyzed on the sequence of the human DNA polymerase ε gene.

As a result of analysis of the 4 specimens with markedly higher mutation rates on the sequence of the human DNA polymerase ε gene, L952S, a mutation that has not been known so far as a mutation that causes hypermutated cancer, was detected from 1 specimen (the specimen indicated with D in Figure 1). From the specimens indicated with A and B in Figure 1, V411L and P286R, mutations that have been already known as mutations that cause hypermutated cancer, were detected. Moreover, the specimen indicated with C in Figure 1 (which has mutation in mismatch repair genes) had no mutation in the human DNA polymerase ε gene.

## Claims

1. A mutant human DNA polymerase ε according to SEQ ID NO: 1 comprising mutation at amino acid position 952,
wherein the mutation comprises mutation of a leucine residue at the position 952 in the amino acid sequence of human DNA polymerase ε into another amino acid residue, wherein the other amino acid residue is a serine residue.

2. A nucleic acid encoding the mutant human DNA polymerase ε according to claim 1.

3. A nucleic acid complementary to a nucleic acid encoding the mutant human DNA polymerase ε according to claim 1.

4. A fragment of the nucleic acid according to claim 2, comprising position 952 in the amino acid sequence of human DNA polymerase ε according to SEQ ID NO: 1, wherein the fragment is a 20 to 300 nucleotide long nucleic acid.

5. A fragment of the nucleic acid according to claim 3, comprising a sequence complementary to position 952 in the amino acid sequence of human DNA polymerase ε according to SEQ ID NO: 1, wherein the fragment is a 20 to 300 nucleotide long nucleic acid.

6. A method for determining whether a cancer is a hypermutated cancer or not in a human patient with cancer, comprising:
determining whether mutation is present or absent at an amino acid residue at the position 952 in the amino acid sequence of human DNA polymerase ε according to SEQ ID NO:1 derived from a cancer cell taken from the human patient using a sample comprising one or more components derived from the cancer cell,
wherein the presence of mutation at the amino acid residue indicates that the cancer is a hypermutated cancer, wherein the mutation at the amino acid reside is L952S.

7. The method according to claim 6, wherein the determination of presence or absence of mutation at the amino acid residue comprises detection of mutation in a nucleotide sequence encoding the amino acid residue at the position 952 in the amino acid sequence of human DNA polymerase ε.

8. The method according to any one of claims 6 to 7, wherein the cancer is colorectal cancer or rectal cancer.

9. A method for diagnosing hypermutated cancer, comprising:
detecting mutation at an amino acid residue at the position 952 in the amino acid sequence of human DNA polymerase ε according to SEQ ID 1 derived from a cell taken from a human specimen using a sample comprising one or more components derived from the cell,
wherein if the amino acid residue is mutated, then the human specimen is diagnosed as having hypermutated cancer, wherein the mutation at the amino acid residue is L952S

## Patentansprüche

1. Mutierte menschliche DNA-Polymerase ε gemäß SEQ.-ID Nr. 1, die eine Mutation an der Aminosäureposition 952 umfasst,
wobei die Mutation eine Mutation eines Leucinrests an der Position 952 in der Aminosäuresequenz von menschlicher DNA-Polymerase ε zu einem anderen Aminosäurerest umfasst, wobei der andere Aminosäurerest ein Serinrest ist.

2. Nukleinsäure, die die mutierte menschliche DNA-Polymerase ε nach Anspruch 1 kodiert.

3. Nukleinsäure, die zu einer Nukleinsäure komplementär ist, die die mutierte menschliche DNA-Polymerase ε nach Anspruch 1 kodiert.

4. Fragment der Nukleinsäure nach Anspruch 2, das Position 952 in der Aminosäuresequenz von menschlicher DNA-Polymerase ε gemäß SEQ.-ID Nr. 1 umfasst, wobei das Fragment eine Nukleinsäure der Länge von 20 bis 300 Nukleotiden ist.

5. Fragment der Nukleinsäure nach Anspruch 3, das eine Sequenz umfasst, die komplementär zur Position 952 in der Aminosäuresequenz von menschlicher DNA-Polymerase ε gemäß SEQ.-ID Nr. 1 ist, wobei das Fragment eine Nukleinsäure der Länge von 20 bis 300 Nukleotiden ist.

6. Verfahren zum Bestimmen, ob ein Krebs bei einem menschlichen Patienten mit Krebs ein hypermutierter Krebs ist oder nicht, das Folgendes umfasst:
Bestimmen, ob eine Mutation an einem Aminosäurerest an der Position 952 in der Aminosäuresequenz von menschlicher DNA-Polymerase ε gemäß SEQ.-ID Nr. 1, die aus einer Krebszelle stammt, die aus dem menschlichen Patienten entnommen wurde, vorliegt oder abwesend ist, unter Verwendung einer Probe, die eine oder mehrere Komponenten umfasst, die aus der Krebszelle stammen,
wobei die Gegenwart einer Mutation an dem Aminosäurerest anzeigt, dass der Krebs ein hypermutierter Krebs ist, wobei die Mutation an dem Aminosäurerest L952S ist.

7. Verfahren nach Anspruch 6, wobei die Bestimmung der Gegenwart oder Abwesenheit einer Mutation an dem Aminosäurerest den Nachweis der Mutation in einer Nukleotidsequenz umfasst, die den Aminosäurerest an der Position 952 in der Aminosäuresequenz von menschlicher DNA-Polymerase ε kodiert.

8. Verfahren nach einem der Ansprüche 6 bis 7, wobei der Krebs kolorektaler Krebs oder rektaler Krebs ist.

9. Verfahren zum Diagnostizieren von hypermutiertem Krebs, das Folgendes umfasst:
Nachweisen einer Mutation an einem Aminosäurerest an der Position 952 in der Aminosäuresequenz von menschlicher DNA-Polymerase ε gemäß SEQ.-ID Nr. 1, die aus einer Zelle stammt, die aus einem menschlichen Präparat entnommen wurde, unter Verwendung einer Probe, die eine oder mehrere Komponenten umfasst, die aus der Zelle stammen,
wobei, wenn der Aminosäurerest mutiert ist, bei dem menschlichen Präparat dann diagnostiziert wird, das hypermutierter Krebs vorliegt, wobei die Mutation an dem Aminosäurerest L952S ist.

## Revendications

1. ADN polymérase ε humaine mutante selon SEQ ID NO: 1 comprenant une mutation au niveau de la position 952 d'acide aminé,
dans laquelle la mutation comprend la mutation d'un résidu leucine au niveau de la position 952 dans la séquence d'acides aminés de l'ADN polymérase ε humaine en un autre résidu d'acide aminé, où l'autre résidu d'acide aminé est un résidu sérine.

2. Acide nucléique codant pour l'ADN polymérase ε humaine mutante selon la revendication 1.

3. Acide nucléique complémentaire d'un acide nucléique codant pour l'ADN polymérase ε humaine mutante selon la revendication 1.

4. Fragment de l'acide nucléique selon la revendication 2, comprenant la position 952 dans la séquence d'acides aminés de l'ADN polymérase ε humaine selon SEQ ID NO: 1, le fragment étant un acide nucléique d'une longueur allant de 20 à 300 nucléotides.

5. Fragment de l'acide nucléique selon la revendication 3, comprenant une séquence complémentaire à la position 952 dans la séquence d'acides aminés de l'ADN polymérase ε humaine selon SEQ ID NO: 1, le fragment étant un acide nucléique d'une longueur allant de 20 à 300 nucléotides.

6. Méthode destinée à déterminer si un cancer est un cancer hypermuté ou non chez un patient humain atteint d'un cancer, comprenant :
la détermination de la présence ou de l'absence d'une mutation au niveau d'un résidu d'acide aminé au niveau de la position 952 dans la séquence d'acides aminés de l'ADN polymérase ε humaine selon SEQ ID NO: 1 dérivée d'une cellule cancéreuse prélevée chez le patient humain en utilisant un échantillon comprenant un ou plusieurs composants dérivés de la cellule cancéreuse,
où la présence d'une mutation au niveau du résidu d'acide aminé indique que le cancer est un cancer hypermuté, où la mutation au niveau du résidu d'acide aminé est L952S.

7. Méthode selon la revendication 6, dans laquelle la détermination de la présence ou de l'absence de mutation au niveau du résidu d'acide aminé comprend la détection d'une mutation dans une séquence nucléotidique codant pour le résidu d'acide aminé au niveau de la position 952 dans la séquence d'acides aminés de l'ADN polymérase ε humaine.

8. Méthode selon l'une quelconque des revendications 6 à 7, le cancer étant un cancer colorectal ou un cancer rectal.

9. Méthode destinée au diagnostic d'un cancer hypermuté, comprenant :
la détection d'une mutation au niveau d'un résidu d'acide aminé au niveau de la position 952 dans la séquence d'acides aminés de l'ADN polymérase ε humaine selon SEQ ID NO: 1 dérivée d'une cellule prélevée chez un spécimen humain en utilisant un échantillon comprenant un ou plusieurs composants dérivés de la cellule,
où, si le résidu d'acide aminé est muté, alors le spécimen humain est diagnostiqué comme étant atteint d'un cancer hypermuté, où la mutation au niveau du résidu d'acide aminé est L952S.
